# EUROPEAN PATENT APPLICATION

(11) **EP 4 682 270 A1**
(43) Date of publication of application: **21.01.2026**
(21) Application number: 23921594.0
(22) Date of filing: 12.09.2023
(51) Int. Cl.: C12Q 1/686, C12Q 1/6895

(54) **RELATIVE QUANTIFICATION METHOD FOR GM CROPS USING DIGITAL PCR WITHOUT CERTIFIED REFERENCE MATERIAL**

(30) Priority: 13.03.2023 KR 20230032250
(71) Applicant: Nex Bio Co., Ltd., Daejeon 34520 (KR)
(72) Inventor: PARK, Yangchan, Daejeon 34199 (KR)
(74) Representative: Ipsilon
(86) International application number: PCT/KR2023/013652
(87) International publication number: WO 2024/190981

(57) **Abstract**

The present invention relates to a method for relative quantification of GM (genetically modified) crops using digital PCR without the need for certified reference material (CRM). More specifically, the present invention relates to a primer and probe set composition for digital PCR to have relative quantification of an introduced gene in GM crops without the need for CRM, comprising a first primer and probe set for specific detection of a gene present in single copy or copy number of 2 to 3 in genome of the GM crops; and a second primer and probe set for specific detection of an introduced gene in the GM crops, and also a use of the primer and probe set composition.

## Description

### TECHNICAL FIELD

The present invention relates to a method for relative quantification of GM (genetically modified) crops using digital PCR without certified reference material (CRM). More specifically, the present invention relates to a method for relative quantification of GM crops without the need for unique CRM of each GM crop by selecting a gene present in single copy or 2 to 3 copies within the genome of GM crops and using the gene as a replacement for CRM.

### BACKGROUND ART

Genetically modified organisms (GMOs) are a solution to various problems facing humanity, such as food shortages, environmental changes, and alternative energy development, and research and development have been made on various crops as they can create high added value. Since genetically modified tomatoes were first developed and commercialized, GMOs have been developed for various crops, and are currently expanding to the development of GMOs with values such as medicine and functionality. As the 'Transboundary Movement, etc. of Living Modified Organisms Act' came into effect in 2008, approval for import of GMOs by use became mandatory for each relevant ministry, and measures such as follow-up management were taken at the national level.

Meanwhile, digital PCR is a third-generation PCR (polymerase chain reaction) method, which is a new approach enabling real-time absolute quantification of target genes for detection. Digital PCR is performed by applying each droplet containing a sample gene template prepared to be diluted to an average copy number of 0.5 to 1, an amplification primer, and a fluorescent probe into one well and carrying out emulsion PCR. Subsequently, wells showing a fluorescent signal indicate that a sample with gene copy number of 1 has been applied and amplified to exhibit the signal, and thus counted as "1". On the other hand, wells without a fluorescent signal indicate that a sample with gene copy number of 0 has been applied so that no amplification occurs to exhibit the signal, and thus counted as "0". Accordingly, absolute quantification can be achieved by counting.

While the existing qPCR (quantitative PCR) result analysis method has analog nature, the digital PCR, which is a digital method in which the result signal has a value of "0" or "1", has advantages that it allows the analysis of large samples and various test items at once. In addition to them, accurate absolute quantification can be achieved by allowing absolute quantification of DNA samples by applying a single molecule counting method that does not require any standard curve.

PCR technique is being used for relative quantification of genes that are introduced into GM crops. This technique is a method of quantifying GMO genes by obtaining a standard curve using a certified reference material (CRM) of the GM crop as a subject of analysis and comparing the PCR results of the analyte with the standard curve. However, the aforementioned method faces several challenges. It does not allow the development of relative quantification assays when CRM cannot be securely obtained or is not present. Additionally, there are issues related to varying amplification efficiency which is caused by differences in nucleic acid quality between CRM and analyte.

Under the circumstances, the present invention provides a method for relative quantification of introduced genes in GM crops, which allows quantification of GMO without the need for CRM.

Meanwhile, Korean Patent Application Publication No. 2019-0136647 discloses a 'Method for detection and absolute quantification of the genus *Alexandrium* using digital PCR.' Additionally, Korean Patent Application Publication No. 2001-0100456 discloses a 'Kit for detection of gene-modified organism and PCR primers used therefor', in which primers capable of hybridizing with specific regions of the 35S promoter are used. However, there is no description relating to the 'Method for relative quantification of GM crops using digital PCR without certified reference material' of the present invention, in which a primer set enabling specific detection of a gene present in low copy number (i.e., single copy or 2 to 3 copies) within the plant genome is utilized.

### DETAILED DESCRIPTION OF THE INVENTION

### TECHNICAL PROBLEMS TO BE SOLVED

The present invention is devised under the aforementioned circumstances, and object of the present invention is to provide a method for relative quantification of GM crops without certified reference material (CRM). Specifically, the genome sequence information of each crop was analyzed to screen for a gene that exists in single copy or copy number of 2, and a molecular marker for digital PCR against the selected endogenous gene and introduced gene was designed to perform digital PCR on GM crop samples. As a result, it was found that the endogenous gene selected through the screening could replace CRM. Furthermore, as a result of analyzing the digital PCR results based on the copy number ratio of the introduced gene and endogenous gene, the method according to the present invention showed that, in terms of the incorporation rate of the GMO sample, the error between the theoretical estimate value and the actual measurement value is significantly lower than the conventional real-time PCR method, and the present invention is completed accordingly.

### TECHNICAL MEANS FOR SOLVING THE PROBLEMS

To achieve the object described in the above, the present invention provides a primer and probe set composition for digital PCR to have relative quantification of an introduced gene in GM (genetically modified) crops without certified reference material (CRM), comprising a first primer and probe set for specific detection of a gene present in single copy or copy number of 2 or 3 in genome of the GM crops; and a second primer and probe set for specific detection of an introduced gene in the GM crops.

The present invention further provides a kit for digital PCR to have relative quantification of an introduced gene in GM crops without CRM, comprising the aforementioned primer and probe set composition for digital PCR and reagents for performing an amplification reaction.

The present invention further provides a method for relative quantification of an introduced gene in GM crops without CRM, including performing PCR using the aforementioned primer and probe set composition for digital PCR of the invention.

The present invention still further provides a marker composition for digital PCR to have relative quantification of an introduced gene in GM crops, comprising MEK1 (mitogen-activated ERK kinase 1) gene, or Ppck1 (phosphoenolpyruvate carboxylase kinase 1) gene as an effective component.

### ADVANTAGEOUS EFFECT OF THE INVENTION

The present invention relates to a method which enables relative quantification of GMO without the need for certified reference material (CRM). Since a gene that exists in single copy or copy number of 2 to 3 in the genome of the GM crops is used as a replacement for CRM, the method has an advantage that relative quantification of a GMO analyte previously not having any CRM can be achieved and the problems relating to amplification efficiency and measurement error due to the differences in nucleic acid quality between CRM and GMO analyte can be avoided. As such, it is expected that the method can be advantageously employed for quarantine of GM crops and inspection of food products containing GM crops.

### BRIEF DESCRIPTION OF THE DRAWINGS

Figure 1 is a schematic diagram showing the relative copy number between the introduced gene and single copy endogenous gene (MEK1, Ppck1) in GM maize or GM soybean.
Figure 2 shows the results of quantitative analysis of the introduced gene in GM maize based on real-time PCR. BT11: introduced gene (i.e., target gene), MEK1: endogenous gene.
Figure 3 shows the results of quantitative analysis of the introduced gene in GM soybean based on real-time PCR. RRS: introduced gene (i.e., target gene), Ppck1: endogenous gene.
Figure 4 shows the results of relative quantitative analysis of the introduced gene (BT11) in GM maize based on digital PCR. The right-hand percentage of the box represents the incorporation rate of GM maize powder which has been calculated, based on the copy number ratio, from the results (i.e., concentrations) of the introduced gene and endogenous gene in each test sample.
Figure 5 shows the results of relative quantitative analysis of the introduced gene (RRS) in GM soybean based on digital PCR. The right-hand percentage of the box represents the incorporation rate of GM soybean powder which has been calculated, based on the copy number ratio, from the results (i.e., concentrations) of the introduced gene and endogenous gene in each test sample.

### BEST MODE(S) FOR CARRYING OUT THE INVENTION

To achieve the object described in the above, the present invention provides a primer and probe set composition for digital PCR to have relative quantification of an introduced gene in GM (genetically modified) crops without certified reference material (CRM), comprising a first primer and probe set for specific detection of a gene present in single copy or copy number of 2 to 3 in genome of the GM crops; and a second primer and probe set for specific detection of an introduced gene in the GM crops.

In the present invention, the term "certified reference material (CRM)" refers to a material that is homogeneous and stable, and also has traceability and uncertainty.

With regard to the primer and probe set composition for digital PCR according to the present invention, the GM crops may preferably be maize or soybean, although not limited thereto.

Additionally, with regard to the primer and probe set composition for digital PCR according to the present invention, the gene present in the genome in single copy or copy number of 2 to 3 is a gene to be used as a CRM for relative quantification of GM crops. This gene can be a gene that is present in a plant in single copy or copy number of 2 to 3 and essential for survival of the plant.

In one embodiment of the present invention, the gene present in the genome in single copy or copy number of 2 to 3 can be MEK1 (mitogen-activated ERK kinase 1), or Ppck1 (phosphoenolpyruvate carboxylase kinase 1), but not limited thereto.

In addition, with regard to the primer and probe set composition for digital PCR according to the present invention, the first primer and probe set is a molecular marker targeting an endogenous gene, which is used as a replacement for CRM, and it is preferably a primer consisting of the nucleotide sequences of SEQ ID NOs: 1 and 2 and a probe consisting of the nucleotide sequence of SEQ ID NO: 3; or a primer consisting of the nucleotide sequences of SEQ ID NOs: 7 and 8 and a probe consisting of the nucleotide sequence of SEQ ID NO: 9, but not limited thereto.

In addition, with regard to the primer and probe set composition for digital PCR according to the present invention, the second primer and probe set is a molecular marker targeting an introduced gene in GM crops, and it is preferably a primer consisting of the nucleotide sequences of SEQ ID NOs: 4 and 5 and a probe consisting of the nucleotide sequence of SEQ ID NO: 6; or a primer consisting of the nucleotide sequences of SEQ ID NOs: 10 and 11 and a probe consisting of the nucleotide sequence of SEQ ID NO: 12, but not limited thereto.

In one embodiment of the present invention, the primer consisting of the nucleotide sequences of SEQ ID NOs: 1 and 2 and probe consisting of the nucleotide sequence of SEQ ID NO: 3 target maize-derived MEK1 gene, the primer consisting of the nucleotide sequences of SEQ ID NOs: 4 and 5 and probe consisting of the nucleotide sequence of SEQ ID NO: 6 target introduced gene BT11 in GM maize; the primer consisting of the nucleotide sequences of SEQ ID NOs: 7 and 8 and probe consisting of the nucleotide sequence of SEQ ID NO: 9 target soybean-derived Ppck1 gene; and the primer consisting of the nucleotide sequences of SEQ ID NOs: 10 and 11 and probe consisting of the nucleotide sequence of SEQ ID NO: 12 target introduced gene RRS in GM soybean.

In one embodiment of the present invention, the primer may include an oligonucleotide which consists of a fragment with 15 or more, 16 or more, or 17 or more consecutive nucleotides from sequences within SEQ ID NOs: 1, 2, 4, 5, 7, 8, 10, and 11, depending on the length of each primer sequence. For example, the primer with SEQ ID NO: 1 (which consists of 18 oligonucleotides) may include an oligonucleotide consisting of a fragment with 15 or more, 16 or more, or 17 or more nucleotides from the sequence of SEQ ID NO: 1.

In the present invention, the term "primer" refers to a single-stranded oligonucleotide sequence that is complementary to the nucleic acid strand to be copied, and it can serve as a starting point for the synthesis of a primer extension product. The length and sequence of the primers must be sufficient to allow the initiation of synthesis of the extension product. The specific length and sequence of the primer will depend on the complexity of the desired DNA or RNA target, as well as the conditions under which the primer is used, such as temperature and ionic strength.

As described herein, the oligonucleotides used as primer may also include a nucleotide analogue, such as phosphorothioate, alkylphosphorothioate, or peptide nucleic acid, or it may include an intercalating agent.

In the present invention, the term "probe" refers to a hybridization probe, i.e., a natural or modified monomer or a linear oligomer with linkage which includes deoxyribonucleotides and ribonucleotides and can bind to complementary strands of nucleic acids in sequence-specific manner. The probe of the present invention is an allelic-specific probe, and, due to the presence of polymorphic site in the nucleic acid fragments derived from two members of the same species, it will hybridize with DNA fragments derived from one member but not with fragments derived from the other member. Preferably, the probe is a single strand, and more preferably, a deoxyribonucleotide to achieve maximum efficiency in hybridization, but it is not limited thereto.

In the present invention, the term "hybridization" refers to a process by which complementary single-stranded nucleic acids form a double-stranded nucleic acid. Hybridization may occur between two nucleic acid strands that are either perfectly matched or substantially matched with some mismatches. The complementarity required for hybridization may vary depending on hybridization conditions, particularly temperature.

In one embodiment of the present invention, the probe consisting of the sequences SEQ ID NOs: 3, 6, 9, and 12 may have a fluorescent dye labeled at one end, either the 5' end or 3' end, and a quencher labeled at the other end. Preferably, the fluorescent dye is labeled at the 5' end, and the quencher is labeled at the 3' end. This probe specifically binds to the product amplified by the primer set. As for the fluorescent dye, FAM, VIC, TET, JOE, HEX, CY3, CY5, ROX, RED610, TEXAS RED, RED670, TYE563, BIOTIN, DIGOXIGENIN, and NED may be used, although it is not limited thereto.

The present invention further provides a kit for digital PCR to have relative quantification of an introduced gene in GM crops without CRM, comprising the aforementioned primer and probe set composition for digital PCR; and reagents for performing an amplification reaction.

With regard to the kit according to the present invention, the reagents for performing an amplification reaction may include DNA polymerase, dNTPs, and buffer, but are not limited to these components.

The kit may also include a user manual detailing the optimal conditions for performing the reaction. This manual is a printed document that explains how to use the kit, including instructions for preparing reverse transcription buffer and PCR buffer, as well as the proposed reaction conditions. The manual includes a guideline booklet like pamphlet or leaflet, a label attached to the kit, or explanations printed on the surface of the package containing the kit. Additionally, the manual may include information disclosed or available through electronic media, such as the internet.

The present invention further provides a method for relative quantification of an introduced gene in GM crops without CRM, including performing PCR using the aforementioned primer and probe set composition for digital PCR of the invention.

More specifically, the aforementioned method may also include:
isolating a genomic DNA from a sample of GM crops;
using the isolated genomic DNA as a template and aliquoting a reaction solution containing a DNA polymerase and the primer and probe set composition for digital PCR to each well of a microfluid digital PCR plate having tens of thousands of partitioned cells per well;
amplifying a target nucleic acid sequence in the each well through PCR; and
determining an amount of the target nucleic acid by counting the number of cells with the amplified target nucleic acid,
but the method is not limited thereto.

The method of the present invention includes a step of isolating a genomic DNA from a sample of GM crop. As for the method for isolating a genomic DNA, any method well-known in the pertinent art can be employed, and, for example, the CTAB method or the Wizard prep kit (Promega) can be used. By having the isolated genomic DNA as a template and performing an amplification reaction with the primer and probe set composition for digital PCR according to one embodiment of the present invention, the target sequence can be amplified.

In the present invention, the principle of digital PCR (dPCR) consists of three main steps: partitioning, amplification, and counting. First, for partitioning, a sample containing DNA is aliquoted into a nanoplate which is composed of wells partitioned into tens of thousands of cells, approximately 26,000 cells per well. Subsequently, PCR amplification is performed. Then, number of cells where the target fragment is amplified is counted, and this count, along with the number of cells where the target is not amplified, is used to achieve the absolute quantification.

The relative quantification method of the present invention is a method which involves first identifying an endogenous gene present in the genome of the plant in low copy number, for example, as single copy or 2 to 3 copies, developing a primer set designed to specifically detect the corresponding gene, and using the primer set not only as an internal control but also as a replacement for CRM.

The present invention still further provides a marker composition for digital PCR to have relative quantification of an introduced gene in GM crops, comprising MEK1 (mitogen-activated ERK kinase 1) gene, or Ppck1 (phosphoenolpyruvate carboxylase kinase 1) gene as an effective component.

Because it is found in the present invention that the MEK1 gene, or Ppck1 gene, which have been selected as a replacement for CRM in GM maize and GM soybean, respectively, can function as a CRM for relative quantification, those genes can be utilized as a marker for digital PCR to have relative quantification of introduced genes in GM crops.

Hereinbelow, the present invention is explained in greater detail in view of Examples. However, the following Examples are given only for exemplification of the present invention and it is evident that the scope of the present invention is not limited by those Examples.

### EXAMPLES

### Materials and Methods

### Example 1. GMO sample and extraction of genomic DNA

In the present invention, the relative quantification method was tested on GM maize, and GM soybean. Maize is a crop that produce offspring through cross-pollination, while soybean a crop that produces offspring through self-pollination. The powder samples of GM maize, and GM soybean used in the present invention were purchased from Sigma (Tables 1 and 3). Genomic DNA was extracted from each purchased sample by using the QIAamp PowerFecal Pro DNA Kit (cat. no. ID 51804) (Tables 2 and 4).

**[Table 1]**

| GM maize powder sample | | | |
|---|---|---|---|
| NO | Name | | |
| 1 | ERM-BF412bk | Maize powder_100 % | BT11 maize |
| 2 | ERM-BF412ek | Maize powder_10 % | BT11 maize |
| 3 | ERM-BF412dk | Maize powder_1.0 % | BT11 maize |
| 4 | ERM-BF412ck | Maize powder_0.1 % | BT11 maize |
| 5 | ERM-BF412ak | Maize powder_0 % | BT11 maize |

**[Table 2]**

| Genomic DNA extracted from GM maize powder sample | | | | | | | | |
|---|---|---|---|---|---|---|---|---|
| NO | DNA Name | Conc. (ng/µL) | Volume (µL) | Amount (µg) | 260/280 | 260/230 | µL for 100ng | |
| | | | | | | | DNA | DW |
| 1 | Maize powder_100 % | 234 | 60 | 14 | 1.84 | 2.14 | 0.4 | 0.6 |
| 2 | Maize powder_10 % | 159 | 120 | 19.1 | 1.82 | 2.06 | 0.6 | 0.4 |
| 3 | Maize powder_1.0 % | 190 | 60 | 11.4 | 1.84 | 1.89 | 0.5 | 0.5 |
| 4 | Maize powder_0.1 % | 173 | 60 | 10.4 | 1.85 | 2 | 0.6 | 0.4 |
| 5 | Maize powder_0 % | 202 | 60 | 12.1 | 1.85 | 2.11 | 0.5 | 0.5 |

**[Table 3]**

| GM soybean powder sample | | | | | |
|---|---|---|---|---|---|
| NO | Name | | | Certified value (g/kg) | Uncertainty (g/kg) |
| 1 | ERM-BF410bp | Soybean powder_100 % | GTS40-3-2 Soybean | >985 | |
| 2 | ERM-BF410ep | Soybean powder_10 % | GTS40-3-2 Soybean | 100 | 5 |
| 3 | ERM-BF410dp | Soybean powder_1.0 % | GTS40-3-2 Soybean | 10 | 0.6 |

**[Table 4]**

| Genomic DNA extracted from GM soybean powder sample | | | | | | | | |
|---|---|---|---|---|---|---|---|---|
| NO | DNA Name | Conc. (ng/µL) | Volume (µL) | Amount (µg) | 260/280 | 260/230 | µL for 100ng | |
| | | | | | | | DNA | DW |
| 1 | Soybean powder_100 % | 42.2 | 70 | 3 | 1.95 | 1.03 | 0.5 | 0.5 |
| 2 | Soybean powder_10 % | 32.7 | 140 | 4.6 | 2.13 | 1.19 | 0.6 | 0.4 |
| 3 | Soybean powder_1 % | 24 | 140 | 3.4 | 2.23 | 0.71 | 0.8 | 0.2 |

### 2. Preparation of primer and probe set for digital PCR

For the qualitative and quantitative analysis of GM maize BT11, primers and probes for detecting endogenous and target genes (i.e., introduced genes) were designed with reference to NCBI Reference Sequence: NM_001371620.1, as detailed in the following Table 5. Similarly, for the qualitative and quantitative analysis of GM soybean RRS, primers and probes for detecting endogenous and target genes were designed with reference to GenBank accession no. DQ657244.1, as detailed in Table 6 that is given below.

**[Table 5]**

| Primers and probes for detecting endogenous and target genes for qualitative and quantitative analysis of GM maize | | | | |
|---|---|---|---|---|
| Name | 5'→3' | Dye | GC % | Tm (°C) |
| MEK1_F1B | TGGGTGGGCACATTATTT (SEQ ID NO: 1) | | 44 | 53.0 |
| MEK1_R1B | AACAACCCCTAACATCCT (SEQ ID NO: 2) | | 44 | 52.0 |
| MEK1_T1B | ACAGCTGACACACCATGTAC (SEQ ID NO: 3) | FAM-BHQ1 | 50 | 58.4 |
| BT11_F1A | AAAAGACCACAACAAGCCGC (SEQ ID NO: 4) | | 50 | 58.2 |
| BT11_R1A | CAATGCGTTCTCCACCAAGTA CT (SEQ ID NO: 5) | | 48 | 62.9 |
| BT11_T1A | CGACCATGGACAACAACCCA AACATCA (SEQ ID NO: 6) | FAM-BHQ1 | 48 | 68.2 |

**[Table 6]**

| Primers and probes for detecting endogenous and target genes for qualitative and quantitative analysis of GM soybean | | | | |
|---|---|---|---|---|
| Name | 5'→3' | Dye | GC % | Tm (°C) |
| PpcK1_F1A | TGGAGTGAAGTAATGCATTGC (SEQ ID NO: 7) | | 43 | 57.5 |
| PpcK1_R1A | ATTTTACTCCCACTGCTGCC (SEQ ID NO: 8) | | 50 | 58.4 |
| PpcK1_T4A | TTGTACTACCGTGCCATTTTG CTAAGT (SEQ ID NO: 9) | FAM-BHQ1 | 41 | 65.3 |
| RRS_F1A | CCTTTAGGATTTCAGCATCAG | | 46 | 63.6 |
| | TGG (SEQ ID NO: 10) | | | |
| RRS_R1A | GACTTGTCGCCGGGAATG (SEQ ID NO: 11) | | 61 | 58.4 |
| RRS_T1A | CGCAACCGCCCGCAAATCC (SEQ ID NO: 12) | FAM-BHQ1 | | 63.6 |

### Example 1. Quantitative analysis of introduced gene in GM crops using real-time PCR

Quantitative analysis was performed using real-time PCR with the extracted genomic DNA and the primer and probe set prepared in the above. The experiment involved preparing a reaction solution containing 20 ng of template DNA, 4.8 µL of each primer and probe, 10 µL of a polymerase mixture containing dNTPs, and 24.2 µL of RNase-free water. The reaction was conducted using the CFX96^{™} Real-Time PCR Detection System.

**[Table 7]**

| Reaction conditions for real-time PCR | | | |
|---|---|---|---|
| Step | Temp | Time | |
| Initial heat activation | 95°C | 15 min | |
| Denaturation | 95°C | 15 sec | 40 cycles |
| Annealing | 57°C | 30 sec | |
| Extension | 72°C | 1 min | |
| Final extension | 72°C | 5 min | |

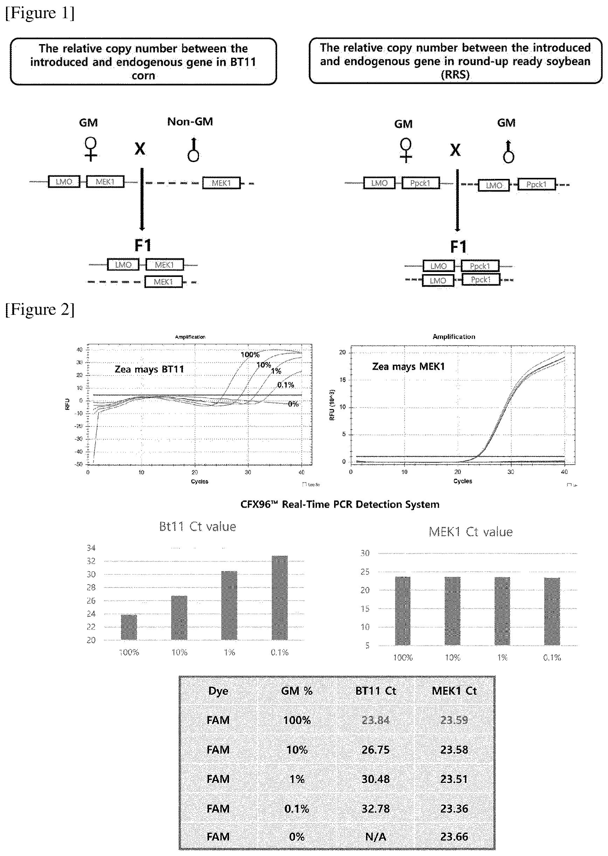

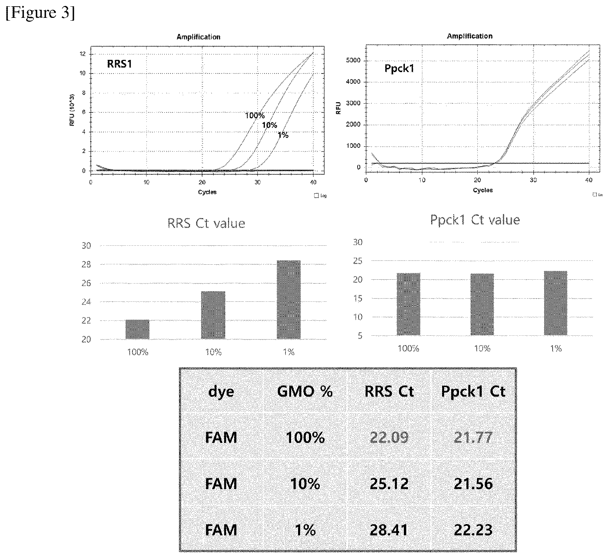

The results showed, as illustrated in Figures 2, and 3, that as the incorporation rate of GM crops in the samples decreased, the Ct values for the target introduced genes (BT11 for GM maize, and RRS for GM soybean) increased. In contrast, it was found that the Ct values for the endogenous genes (MEK1 and Ppck1) remained unchanged.

### Example 2. Quantitative analysis of introduced gene in GM crops using digital PCR

Relative quantification by digital PCR was performed using the same sample and primer/probe set as employed in Example 1. Digital PCR was conducted with QIAcuity device of Nanoplate 26K 24-well plate type, and results were analyzed with the QIAcuity Software Suite 1.2.18 program. The reaction solution used for the digital PCR reaction is shown in the following Table 8, and the amplification conditions were the same as those detailed in Table 7.

**[Table 8]**

| Composition of reaction solution for digital PCR | | | |
|---|---|---|---|
| 26K Nanoplate reaction setup | Singleplex | | 1X (µL) |
| | 4X dPCR probe MM | | 10 |
| | 10X Primer mix | Forward primer (20 pmole) | 0.8 |
| | | Reverse primer (20 pmole) | 0.8 |
| | | Probe (10 pmole) | 0.4 |
| | RNase-free water | | 23.2 |
| | Template DNA | | 2 |
| | Total | | 40 |

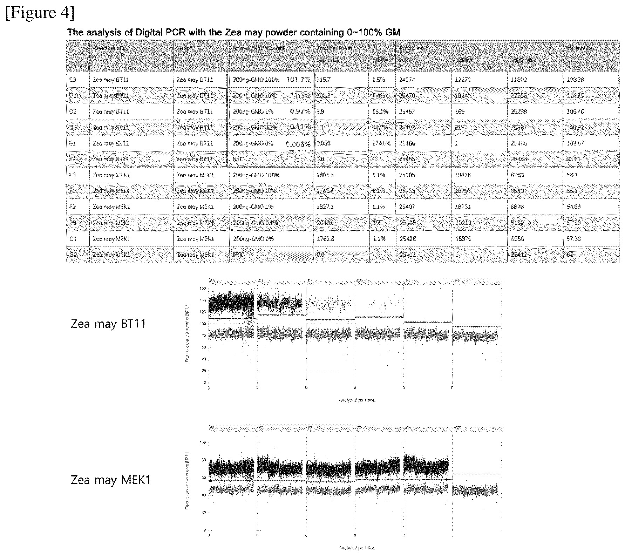

As a result of the analysis, it was found that, the concentration of BT11 gene as the introduced gene decreased with the GMO incorporation rate in GM maize sample, while the concentration of the endogenous gene MEK1 remained unchanged regardless of GMO incorporation rate (Figure 4). As indicated in Figure 1, the copy number ratio of the introduced gene to the endogenous gene in maize is 1 : 2. As such, based on the results (i.e., concentration values) for the introduced gene and endogenous gene in a sample containing 1% GM maize powder, the calculated incorporation rate of GM maize powder was 0.974% ((8.9/1827.1) × 2 × 100), indicating that the theoretical value and the actual value differ by only 2.6%.

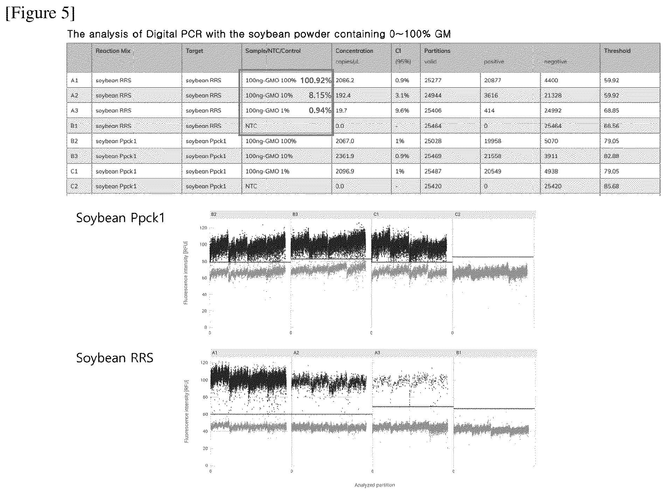

Similarly, the results for GM soybean sample exhibited a trend similar to that observed for GM maize. The concentration of RRS as the introduced gene decreased with the GMO incorporation rate, while the concentration of the endogenous gene Ppck1 remained unchanged regardless of the GMO incorporation rate (Figure 5). Furthermore, based on the 1 : 1 copy number ratio of the introduced gene to the endogenous gene in soybean (Figure 1), the incorporation rate of GM soybean powder in a sample containing 1% GM soybean powder was calculated to be 0.94% ((19.7/2096.9) × 100), indicating that the theoretical value and the actual value differ by 6.1%.

Based on the results given above, it was recognized that the MEK1 gene, or Ppck1 gene selected to replace CRM in GM maize, or GM soybean can function as a certified reference material (CRM) for relative quantification.

## Claims

1. A primer and probe set composition for digital PCR to have relative quantification of an introduced gene in GM (genetically modified) crops without certified reference material (CRM), comprising a first primer and probe set for specific detection of a gene present in single copy or copy number of 2 to 3 in genome of the GM crops; and a second primer and probe set for specific detection of an introduced gene in the GM crops.

2. The primer and probe set composition for digital PCR according to Claim 1, wherein the GM crop is maize, or soybean.

3. The primer and probe set composition for digital PCR according to Claim 1, wherein the gene present in single copy or copy number of 2 in genome is MEK1 (mitogen-activated ERK kinase 1) gene, or Ppck1 (phosphoenolpyruvate carboxylase kinase 1) gene.

4. The primer and probe set composition for digital PCR according to Claim 1, wherein the first primer and probe set is a primer consisting of the nucleotide sequences of SEQ ID NOs: 1 and 2 and a probe consisting of the nucleotide sequence of SEQ ID NO: 3; or a primer consisting of the nucleotide sequences of SEQ ID NOs: 7 and 8 and a probe consisting of the nucleotide sequence of SEQ ID NO: 9.

5. The primer and probe set composition for digital PCR according to Claim 1, wherein the second primer and probe set is a primer consisting of the nucleotide sequences of SEQ ID NOs: 4 and 5 and a probe consisting of the nucleotide sequence of SEQ ID NO: 6; or a primer consisting of the nucleotide sequences of SEQ ID NOs: 10 and 11 and a probe consisting of the nucleotide sequence of SEQ ID NO: 12.

6. A kit for digital PCR to have relative quantification of an introduced gene in GM crops without CRM, comprising the primer and probe set composition for digital PCR of Claim 1 and reagents for performing an amplification reaction.

7. The kit according to Claim 6, wherein the reagents for performing an amplification reaction include a DNA polymerase, dNTPs, and a buffer.

8. A method for relative quantification of an introduced gene in GM crops without CRM, including performing PCR using the primer and probe set composition for digital PCR of Claim 1.

9. The method according to Claim 8, wherein it includes:
isolating a genomic DNA from a sample of GM crops;
using the isolated genomic DNA as a template and aliquoting a reaction solution containing a DNA polymerase and the primer and probe set composition for digital PCR of Claim 1 to each well of a microfluid digital PCR plate having tens of thousands of partitioned cells per well;
amplifying a target nucleic acid sequence in the each well through PCR; and
determining an amount of the target nucleic acid by counting the number of cells with the amplified target nucleic acid.

10. A marker composition for digital PCR to have relative quantification of an introduced gene in GM crops, comprising MEK1 (mitogen-activated ERK kinase 1) gene, or Ppck1 (phosphoenolpyruvate carboxylase kinase 1) gene as an effective component.
